# EUROPEAN PATENT APPLICATION

(11) **EP 4 321 207 A1**
(43) Date of publication of application: **14.02.2024**
(21) Application number: 22189429.8
(22) Date of filing: 09.08.2022
(51) Int. Cl.: A61N 1/372, A61B 5/00, A61N 1/39

(54) **MEDICAL DEVICE SYSTEM AND OPERATION METHOD FOR SAME**

(71) Applicant: BIOTRONIK SE & Co. KG, 12359 Berlin (DE)
(72) Inventor: Taff, Brian M., Portland, 97217 (US); von Arx, Jeffrey A., Lake Oswego, 97035 (US); Kraetschmer, Hannes, West Linn, 97068 (US)
(74) Representative: Biotronik Corporate Services SE

(57) **Abstract**

A medical device system for implantation within a patient is disclosed comprising a first implantable device (1) and a second implantable device (2), wherein the second implantable device (2) is configured to be implanted at a shallow region of the patient's body that provides more reliable data/value transfer from the deeply implanted first device (1) to an external device (3). The first implantable device (1) has a first measurement unit (126) configured to record at least one bodily parameter, a first processor (120) configured to process the recorded values of the at least one bodily parameter and a first communication unit (128), wherein the second implantable device (2) has a second data memory and a second communication unit configured to receive recorded values of the at least one bodily parameter and/or data produced by the first processor (120) based on the recorded values, wherein the second data memory is configured to store received recorded values and/or data and the second communication unit is configured to transmit said recorded values and/or data to the external device (3). A respective operation method of such system is provided, as well.

## Description

The present invention is generally directed to a medical device system for implantation within a patient comprising at least one implantable medical device as well as an operation method of such system.

Nowadays, there is a great variety of implantable medical devices, for example, pacemakers, implantable cardioverter defibrillators (ICDs), implantable cardiac monitors, or spinal cord stimulation devices.

One important example of an implantable medical device is an implantable intracardiac pacemaker (also known as implantable leadless pacemaker - ILP) which is a well-known miniaturized medical device that is entirely implanted into a heart chamber of a patient, either a ventricle or an atrium. ILPs are considered the future of cardiac pacing. Intracardiac pacemakers are used, for example, for patients who suffer from a bradycardia, with a heart that beats too slow to fulfill the physiological needs of the patient. Intracardiac devices may apply electrical stimulation in the form of pulses to the heart to generate a physiologically appropriate heart rate and/or in the form of fast pacing or shocks for cardioversion or defibrillation to restore a more normal heart rhythm. Due to the highly restricted device size, such device has a small battery capacity.

Implantable medical devices are configured to communicate recorded values of the patient's health condition (recorded bodily values) and data derived therefrom as well as therapy data to allow assessment of the patient's health condition and provided therapy, for example during a follow-up. This is helpful for determination of further therapy settings and realization of optimal patient care. For communication of such data far-field RF communication is common in implantable medical devices today. Most use either the MICS band at 403 MHz, ISM at 900 MHz, or BLE at 2.4 GHz. All are suitable for shallow implants such as conventional pacemakers, ICDs or spinal cord stimulation devices. However, far-field RF communications is generally not suitable for deeply implanted medical devices such as ILPs because the RF signals cannot propagate through that much tissue without considerable path loss.

Medicare reimbursement for remote pacemaker checkups in the US is significantly higher when a health care practitioner (HCP) examines a patient electrocardiogram (ECG, a device recorded rhythm strip). Therefore, being able to send an ECG recording from a deep implant such as an ILP is essential not only for optimal patient care, but for adequate remote follow-up reimbursement.

Accordingly, there is the need for a system providing data communication for medical devices which are located at deeper regions of the patient's body and a respective operation method.

The above problem is solved by a system comprising the features of claim 1, an operation method comprising the features of claim 8 as well as a respective computer program product and a respective computer readable data carrier.

In particular, the above problem is solved by a medical device system for implantation within a patient comprising a first implantable device and a second implantable device, wherein the second implantable device is configured to be implanted at a shallow region of the patient's body. The first implantable device has a first measurement unit configured to record at least one bodily parameter, a first processor configured to process the recorded values of the at least one bodily parameter and a first communication unit, wherein the second implantable device has a second data memory and a second communication unit configured to receive recorded values of the at least one bodily parameter and/or data produced by the first processor based on the recorded values, wherein the second data memory is configured to store received recorded values and/or data, wherein the second communication unit is further configured to transmit said recorded values and/or data to an external device, wherein the communication between the first communication unit and the second communication unit is an intra-body communication using ultrasonic signals and/or electric signals.

The medical device system incorporates two medical devices, wherein at least one device, the second implantable device is one configured to be implanted shallowly (i.e. depth with regard to the outer skin of the patient < 4 cm) in the body. Shallow implants may take on a variety of types (nominally pacemakers, ICDs, and cardiac monitors) and can vary in form factor (pacemakers = thin and flat with volumes of 5-15 cc; ICDs thicker but still flat with volumes 10-20 cc; cardiac monitors = long and thin with volumes of 1-5 cc). The pacemakers and ICDs are preferably subcutaneous or submuscular because, full support of brady- and tachy-remediation for 10-15 year service times forces them to need chips, components, and power sources, that simply make them too large to be anything but subcutaneous (or submuscular) without creating unnecessary complication. In other words, they can't be anything but subcutaneous/submuscular devices and therefore there's not much that is special about them that makes the fact that they are subcutaneous/submuscular an asset. Their implantation also may demand that a surgical pocket be cut into the patient's chest which is actually considered to be not particularly desirable. As for cardiac monitors, their slender format makes them easier to implant in outpatient settings compared to pacemakers and ICDs. To implant them in a manner other than subcutaneous/submuscular would add procedural and patient wellbeing risks that are unnecessary.

The other implantable medical device (first implantable device) may be implanted shallowly or deeply in the body ("deeply" means depth with regard to the outer skin of the patient >= 4 cm). The first implantable device (e.g. an ILP implanted in the heart, or a pulmonary arterial pressure sensor in the pulmonary artery) sends and receives signals to/from the second implantable device via intrabody communications. The intrabody communications (IBC) can be acoustic (ultrasound) or galvanic (conducted electrical pulses in the body), or some other mode. Both acoustic and galvanic communication offer excellent signal propagation in the body with relatively low loss. Best practices acoustic methods may be/leverage ultrasound-based tactics and likely employ a piezoelectric component resident wholly within or incorporated as part of the implant housing. Subject to appropriate electrical stimulation, the piezoelectric component (perhaps best implemented as a disk attached to the battery header) may change dimensions to transmit sound waves through/from the implant housing and into the patient's anatomy. Said acoustic waves may then be detectable by the second implantable device as what amount to localized pressure waves. As for conducted electrical pulse-based communication, may - in a preferred embodiment - leverage paced output within the refractory periods of the pacemaker. Pace output in refractory periods would avoid any risks for unwanted heart stimulation outside of nominal therapy support. As a further safeguard the amplitude of the output in such refractory periods may leverage low-amplitude paces that would avoid exceeding pacing capture thresholds (if they were applied to patient tissue at times outside the refractory periods). In both cases (acoustic and galvanic) the signal is modulated to encode the transmitted data. One modulation technique uses OOK (On Off Keying) to encode the data. Here the presence or absence of the acoustic/galvanic signal encodes a logical 1 or 0. Other modulations techniques such as ASK (amplitude shift keying), FSK (frequency shift keying) or QAM (Quadrature amplitude modulation) may also be used.

The second implantable device may be a hub-like bio-monitor. Alternatively, the second implantable device may be a subcutaneous ICD (S-ICD). The system may comprise an ILP and an S-ICD.

The first device (deep implant or implants) communicates by IBC with the second device (shallow implant or implants) using ultrasonic or electric signals. The second implantable device, i.e. its second communication unit forms a hub because it relays information (recorded values/data) out of the body, for example by far field RF to an external device, for example a patient monitoring device (e.g. a home monitoring device or an "off the shelf' smartphone). Additionally, the second communication unit of the second implantable device may relay information it receives from outside the body to the first implantable device by IBC. Further, the system may comprise two or more first implantable devices which may be implanted within the same patient's body. Each of them may be configured to communicate with one second implantable device. In one embodiment, the system may comprise two or more second implantable devices.

The first communication unit of the first implantable device may comprise a sender or a transceiver, wherein the second communication unit comprises a transceiver. The sender or transceiver of the first communication unit and the receiver part of the transceiver of the second communication unit are configured to communicate via IBC. There may be a variety of data encoding schemes that could be paired with any specific physical embodiment of the IBC. For example, OOK, Binary Phase-Shift Keying (BPSK), frequency modulation and others are readily approachable. Nominally, phase-shift keying techniques may be a suitable encoding scheme as there are few elements within the patient's body that can serve to introduce phase noise and thus tend to facilitate access to reasonably high signal noise ratios (SNR) without instating excessive comparable penalties in the power/bit of data sent.

The transmitter part of the transceiver of the second communication unit may communicate with the respective receiver of the external device using a communication technique wirelessly via the air using electromagnetic waves, for example, MICS (Medical Implant Communication Service), EDGE, EV-DO, Flash-OFDM, GPRS, HSPA, LoRaWAN, RTT, UMTS, Narrowband IoT, Bluetooth, BLE (Bluetooh Low Energy), WLAN (WiFi), ZigBee, NFC, LTE, Wireless USB, Wibree (BLE), Ethernet or WiMAX in the radio frequency region, or IrDA or free-space optical communication (FSO) in the infrared or optical frequency region. Accordingly, the respective communication protocols, often with the same name (i.e. a system of rules that allows two or more entities of a communications system to transmit information via an kind of variation of a physical quantity, defining rules, syntax, semantics and synchronization of communication and possible error recovery methods and being implemented by hardware, software or a combination of both) or cooperating protocols (protocol suites) may be used, for example TCP/IP protocol suite (e.g. IPv6, TCP, UDP, SMTP, HTTP/2) also with TLS or SSL, IPX/SPX, X.25, AX.25, ebXML, Apple Talk, Bluetooth protocols (e.g. BR/EDR), Bluetooth 4.0 (BLE), ZigBee protocol, NFC protocol, IEEE 802.11 and 802.16 protocols, etc.

The first implantable device has a first measurement unit configured to record at least one bodily parameter, a first processor configured to process the recorded values of the at least one bodily parameter and a first communication unit. The bodily parameter may, for example, comprise a vital sign of the patient's body such as the heart rate, blood pressure, blood sugar, ECG, quality of heart wall contractility, breathing rate, breathing tidal volume, activity, temperature, heart sounds, blood oxygen saturation, the evoked response from a nerve, a recording from a nerve or otherwise. From the measured bodily signals the processor may determine additional data such as the percent of effective pacing output, degree of observed AV synchrony, the regularity of viable cardiac conduction, arrhythmic conditions, apnea hypopnea index, worsening heart failure, or otherwise. In one embodiment, the first measurement unit is configured to sense the patient's native intracardiac electrical signaling and use this sensed information (at least in part) to determine, for example, the heart rate, PR-interval, QT-interval, ST-interval, P-wave and T-wave durations. In addition to the sensed native intracardiac electrical signaling, the first measurement unit additionally may detect pacing output (e.g. its start time position and its duration as well as its frequency) or other treatment signals from the second implantable device. Further, the first measurement unit may be adapted to determine the impedance to derive cardiac tissue properties. The first measurement unit may also sense multiple signals indicative of multiple contractions of the ventricle and determine the ventricular contraction rate from the multiple signals. Sensed signals may include, for example, far-field R-waves and/or heart sounds. The embodiment where the first measurement unit collects ECG data and/or IEGM data and the first communication unit sends these data to the second communication unit and of the second implantable device and this device further passes these data to an external device, e.g. a home monitoring computer, is particularly important because reimbursement for remote pacemaker/ILP follow-ups is significantly higher when an ECG or IEGM strip is transmitted and read by the remote HCP. The first implantable device may have a first data memory for storing recorded values and data produced by the first processing unit.

The external device may be a computer configured for home-monitoring. Thus, the external device and/or the computer may be a smartphone or another patient device, e.g. a Biotronik CardioMessenger.

The second implantable device has a second data memory and a second communication unit configured to receive recorded values of the at least one bodily parameter and/or data produced by the first processor based on the recorded values, wherein the second data memory is configured to store received recorded values and/or said data. Within the second data memory, the second implantable device stores the data/recorded values it receives before forwarding it on to the external device. Immediately after receipt or at some later point the second communication unit of second implantable device (hub) then forwards the data/recorded values to the external device, e.g. a home monitoring infrastructure. This is important because it allows the first implantable device to have minimal memory because it can transmit recorded values/data (e.g. ECG or IEGM data) to the second implantable device (hub) for storage. Further, it allows the two transmissions steps to be asynchronous. This is particularly important because the second implantable device (hub) may not be in range of an external device when it receives recorded values/data from the first implantable device (deep implant). Accordingly, the second data memory allows the second implantable device to store and forward the data/recorded values later. Additionally, the second implantable device may consolidate many ECG/IEGM data and send them all together when it recognizes that an external device (e.g. a remote monitoring device) is within range.

The second data memory and, if applicable, the first data memory may include any volatile, non-volatile, magnetic, or electrical media, such as a random access memory (RAM), read-only memory (ROM), non-volatile RAM (NVRAM), programmable ROM (e.g. EEPROM), flash memory, or any other memory device. The first and second data memory may save thresholds, values, flags, parameters and conditions which are required by the processor during processing the above and below explained steps and may save recorded values/data as explained above.

As indicated above, the first implantable device comprises a first processing unit. The second implantable device may comprise a processing unit (in the following: second processing unit), as well. With regard to the invention, the first or, if applicable, the second processing unit is generally regarded as a functional unit of the device, that interprets and executes instructions comprising an instruction control unit and an arithmetic and logic unit. The respective processing unit may comprise a microprocessor, a controller, a digital signal processor (DSP), an application specific integrated circuit (ASIC), a field-programmable gate array (FPGA), discrete logic circuitry or any combination thereof. Alternatively, or additionally, the respective processing unit may be realized using integrated dedicated hardware logic circuits, in particular in the case of an ILP due to the small size and extreme power limitation.

The second implantable device may additionally comprise a second measurement unit, for example a sensor for electrical or electromagnetic signals, an accelerometer sensor and/or an impedance sensor, configured to record measurement values of at least one bodily parameter, wherein the second communication unit is configured to transmit to the external device these further recorded values of the at least one bodily parameter determined by the second measurement unit and/or data produced by a second processor based on said further recorded values.

As indicated above, in one embodiment, said recorded values, said further recorded values and/or said data of the first measurement unit and/or of the second measurement unit are ECG data and/or IEGM data.

In the above embodiment, the second implantable device contains its own at least one physiological sensor forming the second measurement unit and it transmits to the external device both the sensed data it receives from the first implantable device and the sensed data it records from its own at least one physiological sensor of the second measurement unit. In another embodiment, the second implantable device records data by its second measurement unit at the same time as it receives the data from the first implantable device (deep implant), resulting in near simultaneous recordings. This is important because it results in time-synchronous recordings. In one embodiment the first implantable device is an ILP, and the physiological signals from the ILP are IEGMs collected using a short vector within the heart, and the second implantable device is an ICM, and the physiological signal it records are ECG data over a large vector several cm from the heart. Both data sets may be transmitted by the second communication unit of the ICM (after receipt of the IEGM data from the ILP) to the external device. The ILP records the exact signal the pacemaker is seeing, while the ICM is recording a signal closer to what a conventional ECG would see. Both data together (time aligned) are of great use clinically when diagnosing arrythmias, device sensing issues, and other problems.

The first implantable device and the second implantable device may comprise electrical circuitry, for example comprising an application specific integrated circuit (ASIC), for realization of the first measurement unit, the first communication unit and the first processor as well as the second communication unit and the second data memory (and the second processor, if applicable), respectively. Further, each implantable device may comprise a power supply such as a battery, and/or a unit for the application of a therapy, for example a generator of an electrical stimulation signal. The first implantable device may comprise a first data memory for storing data and/or a communication module for communication with the external device which is located outside of the patient's body but, at least sometimes so close that it is in communication range with the second implantable device. For each of the first implantable device and the second implantable device applies that the above units/elements may be electrically interconnected with the electrical circuitry of the respective device and thereby with the own power supply, as well. Most elements of the first implantable device and most elements of the second implantable device, respectively, are contained within one separate hermetically sealed housing, i.e. the first housing or the second housing.

However, there may be some elements that are not contained within the hermetically sealed housing(s). Electrodes as well as MICS- and BLE- antennas are not in the housing, and in some devices as communication coils are in the header, which is not the hermetically sealed housing.

In one embodiment, the first communication unit and/or the second communication unit is configured to transmit said recorded values, said further recorded values and said data at a pre-defined fixed time interval or a varying time interval, for example if fulfillment of a pre-defined condition is recognized. In one embodiment, it is important for the communication between the second communication unit and the external device for reliable data/value transfer that the second communication unit is configured to transmit said recorded values, said data and/or said further recorded values only in case the second measurement unit has recognized the pre-defined condition that the external device is located within a communication range of the second communication unit. Further, in one embodiment, for example the first communication unit of the first implantable device sends the physiological values it has recorded from its first measurement unit to the second communication unit of the second implantable device in slower than real-time. This means that seconds worth of IEGM may be slowly sent from the deep/first implantable device to the shallow/second implantable device over the course of many minutes. The second device would assemble them as needed and then share them with home monitoring or external devices (e.g., Programmer) without having the home monitoring or external devices needing to know about this slower IBC data transmission. This slow trickling out of data from the deep implant to the shallow implant may apply for other statistics as well (e.g., counter data, histogram data, and/or trend data) and simply send data when so doing is referenced to a fixed point in time (i.e., so all the data has the same time stamp since the prior follow-up). Whether this slower than real-time is used to send the IEGM or to send the statistics, the slower transmission may assist in extending device longevity b/c the IBC transmission burden could potentially tax the in-device battery less.

Slower data rates generally ease peak power constrains, which helps with battery longevity. Slower than real-time data transmission requires that the first implantable device comprises a first data memory (i.e. a data buffer) to store the portion of the recorded values that has not yet been sent. This embodiment may be especially important for the smallest first implantable device (such as a pulmonary arterial blood pressure sensor) which may only have a battery with a few mAh capacity. In another embodiment, the deep implant doesn't start to send the physiological values it is recording until the time point after it has recorded the data values and stored it in its first data memory. Then, or at some later time point it transmits the recorded values to the second communication unit of the second implantable device. This embodiment may be especially important for systems in which the IBC transmissions may interfere with the measurements of bodily values. By separating in time the recording of bodily values from the IBC transmissions, the interference is mitigated. For example, if the first implantable device is an ILP, and the IBC uses galvanic communications, then the electrical pulses used for galvanic communications would potentially add noise to the ECG or IEGM heart signal recording. By separating these two steps in time, clean and noise free bodily value recordings can be obtained.

Additionally, it is advantageous for further processing and/or display of the recorded values and data that the first communication unit and/or the second communication unit is configured to transmit said recorded values, said data and/or said further recorded values together with a respective time stamp of the time and/or date of recording. Accordingly, the data and recorded values determined by different devices may be assigned to each other based on the time stamp stored for the respective data/values. The at the same time recorded values and/or data of such values received from the first and/or the second implantable device may then be analyzed and displayed synchronously at the external device.

Some HCPs want to see patients in person rather than remotely during a follow-up. Even for these HCPs the above system can ease their workflow. In one embodiment, the second implantable device stores the recorded values/data it receives from the first implantable device until the next patient follow-up appointment. At that point an HCP can interrogate and download stored recorded values and data such as ECG/IEGM data as well as device data from the second implantable device rather than directly from the first implantable device. It is much easier and faster to communicate with a shallow first implantable device (the hub) rather than having to communicate directly with the deeply within the patient's body located second implantable device. Such shallowly implanted first device can be interrogated wirelessly from across a room, whereas deeply implanted second device generally have limited data rates due to peak power constraints and require a transmit/receive transducer close to the skin. For these reasons a shallowly implanted first device paired with a deeply implanted second device offers advantages even for HCP who choose not to utilize remote monitoring and remote follow ups.

The above problem is solved analogously by an operation method of a medical device system for implantation within a patient comprising a first implantable device and a second implantable device, wherein the second implantable device is configured to be implanted at a shallow region of the patient's body, wherein a first measurement unit of the first implantable device records at least one bodily parameter, a first processor processes the recorded values of the at least one bodily parameter, wherein a second communication unit of the second implantable device receives recorded values of the at least one bodily parameter and/or data produced by the first processor based on the recorded values transmitted from the first communication unit, wherein a second data memory of the second implantable device stores received recorded values and/or data, wherein the second communication unit transmits said recorded values and/or data to an external device, wherein the communication between the first communication unit and the second communication unit is an intra-body communication using ultrasonic signals and/or electric signals.

In one embodiment of the method a second measurement unit of the second implantable device records values of at least one bodily parameter, wherein the second communication unit transmits to the external device further recorded values of the at least one bodily parameter by the second measurement unit and/or data produced by a second processor based on said further recorded values.

In one embodiment of the method said recorded values, said further recorded values and/or said data are ECG data and/or IEGM data.

In one embodiment of the method the first communication unit and/or the second communication unit transmits said recorded values, said further recorded values and said data at a pre-defined fixed time interval or a varying time interval, for example if fulfillment of a pre-defined condition is recognized.

In one embodiment of the method, the second communication unit transmits said recorded values, said data and/or said further recorded values only in case the second measurement unit has detected that the external device is located within a communication range of the second communication unit.

In one embodiment of the method, the first communication unit and/or the second communication unit transmits said recorded values, said data and/or said further recorded values together with a respective time stamp of the time and/or date of recording.

In one embodiment, the second implantable device processes the data from the first implantable device prior to sending it to the external device. Processing the data may include encrypting it, compressing it, and/or prioritizing it so that the most critical data is sent first.

An embodiment where the 2^{nd} device encrypts the data before sending it to the external device is particularly important. Encrypting patient data before transmitting it outside the body is required for many applications. Encryption can be computationally expensive and therefore demanding on the battery. Since the first implantable device generally has a smaller battery and is deeper in the body and therefore less easily replaced when the battery reaches end of life, it is better to shift as much of the computationally expensive processing, such as encryption, to the second implantable device.

The embodiments described for the system apply to the above methods, as well, causing the analogous advantages. It is referred to above embodiments of the system in this regard.

The above method is, for example, at least partly realized as a computer program (to be executed at or within the first and/or second implantable device, in particular utilizing their processors) which is a combination of above and below specified (computer) instructions and data definitions that enable computer hardware or a communication system to perform computational or control functions and/or operations, or which is a syntactic unit that conforms to the rules of a particular programming language and that is composed of declarations and statements or instructions needed for an above and below specified function, task, or problem solution.

Furthermore, a computer program product is disclosed comprising instructions which, when executed by a processor, cause the processor to perform the steps of the above defined method. Accordingly, a computer readable data carrier storing such computer program product is described.

The present invention will now be described in further detail with reference to the accompanying schematic drawing, wherein
- Fig. 1: shows an exemplary medical device system after implantation within a human patient's body comprising an ILP and an S-ICD, and
- Fig. 2: depicts a functional block diagram of the ILP of the embodiment shown in Fig. 1,
- Fig. 3: depicts a flow chart of an inventive operation method.

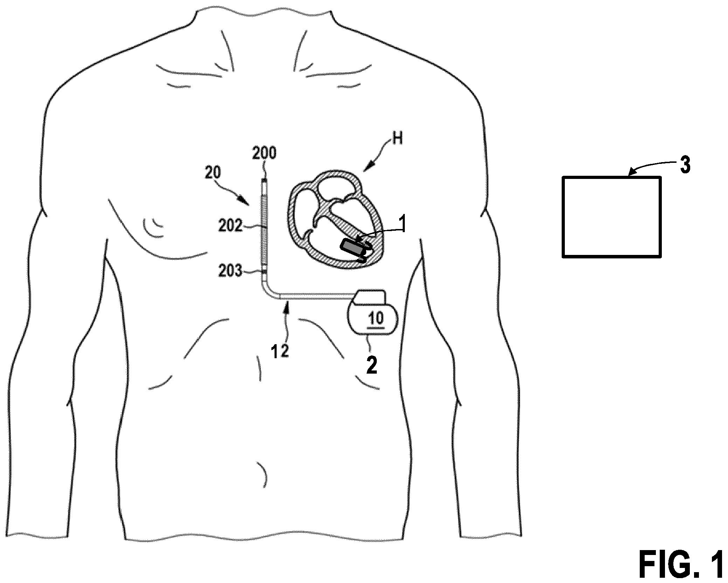

Fig. 1 shows an embodiment of a medical device system implanted in a human patient's body. The system comprises an implantable leadless pacemaker (ILP) 1 as a first device deeply implanted within one, e.g. the right, ventricle of the heart H. Further, the system comprises a subcutaneously implanted, i.e. shallowly implanted, cardioverter defibrillator (S-ICD) 2 as a second device with a housing 10 and an electrode lead 12 connected to the housing 10. In particular, the housing 10 of the S-ICD 2 is shallowly located within the patient's body.

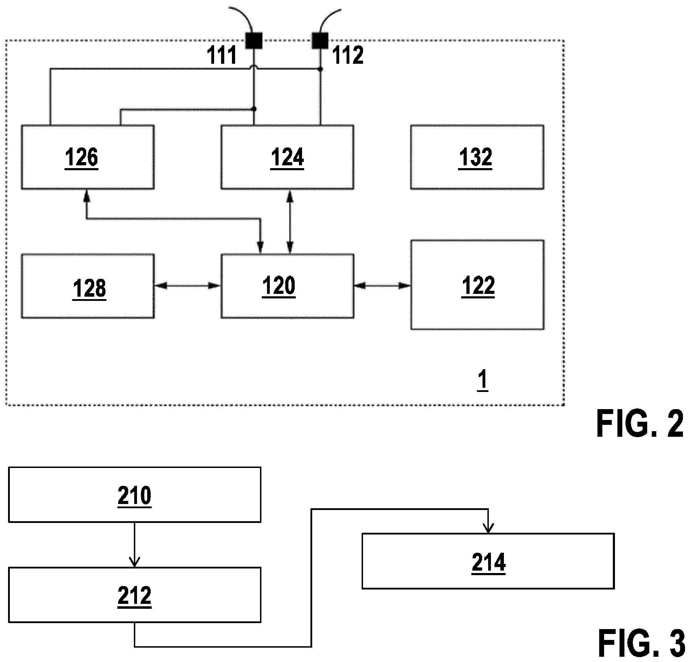

The ILP 1 comprises one or more electrodes 111, 112 (see Fig. 2) which belong to a first measurement unit 126 for sensing electric signals of the heart H. Further, the one more electrodes 111, 112 may be used for application anti-tachycardia pacing (ATP) therapy or other cardiac therapy. The detected electric signals are transmitted to a first processor 120 accommodated within the ILP 1. The first processor 120 may analyze the electric signals of the heart (e.g. IEGM signals) and thereby may determine, for example, the patient's cardiac rate. The first processor 120 is further adapted to generate and deliver signals for standard bradycardia remediation pacing therapies and/or also potentially for ATP therapy to the one or more electrodes 111, 112 of the ILP 1 using a first signal generator unit 124.

Fig. 2 shows a functional block diagram of the ILP 1 (Fig. 1). ILP 1 includes the first processor 120, a first data memory 122, the first signal generator unit 124, the first measurement unit 126, a first communication unit 128, and a first power source 132, wherein all elements are accommodated within and hermetically sealed by the housing of ILP 1. The first power source 132 may include a battery, e.g., a rechargeable or non-rechargeable battery. The first power source 132 is connected to all electrical units of the ILP1 (not shown) to provide electrical energy. Units of the present disclosure may include any discrete and/or integrated electronic circuit components that implement analog and/or digital circuits capable of producing the functions attributed to the units herein. For example, the units may include analog circuits, e.g., amplification circuits, filtering circuits, and/or other signal conditioning circuits. The units may also include digital circuits, e.g., combinational or sequential logic circuits, memory devices, etc. The first data memory 122 may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device. The functions attributed to the units herein may be embodied as one or more processors, hardware, firmware, software, or any combination thereof. Depiction of different features as units is intended to highlight different functional aspects and does not necessarily imply that such units must be realized by separate hardware or software components. Rather, functionality associated with one or more units may be performed by separate hardware or software components or integrated within common or separate hardware or software components. First processor 120 may communicate with first data memory 122. First data memory 122 may include computer-readable instructions that, when executed by processor 120, cause the processor 120 to perform the various functions attributed to processor 120 herein. For example, data memory 122 may include pacing instructions and first signal parameters for a first treatment signal, such as a baseline ventricular pacing rate, a baseline ventricular pacing interval and a baseline AV delay.

The first processor 120 may communicate with first signal generator unit 124 and first measurement unit 126. First signal generator unit 124 and first measurement unit 126 are electrically coupled to electrodes 111, 112. As indicated above, first measurement unit 126 may be configured to monitor signals from electrodes 111, 112 to monitor electrical activity of heart H, for example by measuring an IEGM. Further, the first measurement unit 126 may include an accelerometer and/or a pressure sensor. First signal generator unit 124 is configured to deliver electrical stimulation to the ventricle of the heart H via electrodes 111, 112.

First processor 120 may control first signal generator unit 124 to generate and deliver electrical stimulation to the ventricle via electrodes 111,112. Electrical stimulation may include pacing pulses. First processor 120 may control first signal generator unit 124 to deliver electrical stimulation therapy according to one or more ventricular therapy programs including pacing instructions and values, which may be stored in first data memory 122.

First measurement unit 126 may include circuits that acquire electrical signals from the sensor(s). Electrical signals acquired by first measurement unit 126 may include intrinsic cardiac electrical activity, such as intrinsic ventricular and/or intrinsic ventricular cardiac electrical activity. First measurement unit 126 may filter, amplify, and digitize the acquired electrical signals to generate raw digital data. First processor 120 may receive the digitized data generated by first measurement unit 126. In some examples, first processor 120 may perform various digital signal processing operations on the raw data, such as digital filtering. First processor 120 may sense cardiac events based on the data received from first measurement unit 126. For example, first processor 120 may determine ventricular events based on the data received from first measurement unit 126 and/or the present cardiac rate.

ILP 1 may include the housing and fixation tines protruding from the housing, wherein the housing may have a pill-shaped cylindrical form factor in some examples. Fixation tines are configured to connect (e.g., anchor) ILP 1 to heart H. Fixation tines may be fabricated from a shape memory material, such as Nitinol. In some examples, fixation tines may connect ILP 1 to heart H within one of the chambers of heart H. For example, as illustrated herein in Fig. 1, fixation tines or any other fixation mechanism may be configured to anchor ILP 1 to cardiac tissue of the heart H within right ventricle.

The first communication unit 128 may enable ILP 1 to communicate with the second device, the S-ICD 2, via IBC using ultrasonic signals and/or electric signals. The IBC contains transmission circuitry and/or receiver circuitry. The transmission circuitry may contain a digital encoder, a modulator, a mixer, a local oscillator, a band pass filter, and/or an output amplifier. The receiver circuitry may contain a pre-amplifier, a band pass filter, an amplifier, a detector, and/or a decoder.

In the embodiment where the IBC uses electric signals for communication, then the transmission and/or receiver circuitry is connected to electrodes 111 and 112. The electrodes transmit the data as small, modulated currents flowing through the body. The electrodes receive a signal as small, modulated currents flowing into the electrodes from the body. It is important that these signals be sub-threshold, which means of a small enough amplitude and/or a high enough frequency that they do not stimulate any muscle or never fibers. In another embodiment the IBC is connected to dedicated communication electrodes rather than electrodes 111 and 112 (which primarily serve as the therapy electrodes).

In the embodiment where the IBC uses ultrasonic signals for communication, the transmission and/or receiver circuitry is connected to a piezoelectric transducer. When transmitting the electrical signal from the transmission circuitry causes the piezoelectric transducer to vibrate. This vibration launches modulated ultrasonic pressure waves (I.e., ultrasonic sound waves) into the body. Ultrasound is chosen in part so that the patient cannot hear the transmission. When receiving, the piezoelectric transducer picks up ultrasonic pressure waves from the body and converts these into an electrical signal that is passed to the receiver circuitry of the IBC.

The communication between the S-ICD 2 and the ILP 1 may be one-directional (either from ILP 1 to S-ICD2 only or from S-ICD2 to ILP 1 only) or two-directional. The communication may further be used to coordinate/control the therapy of both devices.

The S-ICD 2 comprises within its housing 10 a second processor, a second communication unit, a second power supply and a shock unit. The lead 12 is implanted subcutaneously along the sternum 20 and comprises two detection electrodes 200, 203 as a part of a second measurement unit and a shock coil 202 for application of a cardioversion or defibrillation shock. The detection electrodes 200, 203 detect electric signals of and around the heart H and transmit these signals to the second processor accommodated within the housing 10. The second processor analyzes the electric signals of the heart H (e.g. the ECG signals) and thereby determines the patient's cardiac rate. The second processor is further adapted to generate and deliver signals transmitted to the shock unit which generates at least one cardioversion or defibrillation shock for anti-tachycardia therapy to shock coil 202. The shock unit has to run through a loading/charging time in which the necessary electric voltage is generated for the one or more shocks delivered by the shock coil 202 as shock therapy. The energy for operation of the S-ICD 2 and the shocks is provided by a power source accommodated with in housing 10 of the S-ICD 2.

Regarding the functional units the structure of S-ICD 2 may be similar to the structure of ILP 1 as described above. For example, the S-ICD 2 may have the second processor similar to the first processor 120, a second data memory similar to the first data memory 122, a second signal generating unit formed by the shock unit, and the second measurement unit similar to the first measurement unit 126. Further, the S-ICD 2 may comprise a second communication module similar to the first communication unit 128 for communication with the ILP 1 and an external device 3. The second communication unit may comprise a transceiver configured to receive the signals of IBC provided by the first communication unit 128 of ILP 1 and to communicated with the external device 3 and transmit the recorded values/data received from the ILP 1 and, if applicable, measurement values and further data determined/created by S-ICD 2 to the external device 3. For example, MICS, BLE (Bluetooth Low Energy), WLAN (WiFi) communication technique using the 2.4 GHz or 5 GHz band may be used.

The external device 3 may be a smartphone, a Programmer, or a unit for home monitoring engagements. It comprises (beside the usual components of a smartphone or a computer) a receiver for the signals provided by the S-ICD 2, for example a MICS, BLE or WLAN (WiFi) receiver operating in the 2.4 GHz or 5 GHz band.

The flow chart of Fig. 3 shows an example of an operation method of the system shown in Fig. 1.

In the first step 210, the ILP 1 determines, for example hourly, an IEGM using its first measurement unit 126. The recorded IEGM values are transmitted to the first processor 120. The first processor 120 determines different data from the IEGM, for example the present cardiac rate.

Then, in the second step 212, the recorded IEGM values and the determined data such as the cardiac rate are transmitted to the S-ICD 2 using the communication unit 128 via the above-described IBC. Accordingly, the IEGM values and the data are received by the second communication unit of the S-ICD 2 and stored there in its second data memory. The first and the second step 210, 212 may be repeated several times before the following third step 214 is executed.

In another embodiment, the implant may store the full IEGM ever and instead simply sense and relay it via IBC so that the only location where the full IEGM is stored would be in the second/shallow implant. In other words, the system could effectively skip using 210 and only use 212 and 214

At a pre-defined time point during the day or if the S-ICD 2 detects that the external device 3 is within its communication range, the second communication unit of S-ICD 2 transmits the IEGM values and the other data determined by the processor 120 such as the cardiac rate received from the ILP 1 to the external device 3 (step 214). The communication may be provided by MICS, BLE, WLAN (WiFi) in the 2.4 GHz or 5 GHz band, for example. Accordingly, the external device 3 receives these values/data by its respective communication unit and stores them in its data memory. In one embodiment, the recorded IEGM values and data may be transmitted from the ILP 1 to S-ICD 2 and finally to the external device with a time stamp (comprising date and time) of their recording. In one embodiment, the S-ICD 2 determines some further values, e.g. ECG values and transmits them (if applicable with a time stamp of their recording) together with the IEGM values and data of the same time interval to the external device 3.

The above system and method are an active implantable medical device system with respective operation method consisting of at least two implants, one (the S-ICD 2) serving as a communications hub for another device (ILP 1) implanted deeper in the patient's body. Such a system offers two advantages, namely, the hub collects and stores patient's bodily data that is streamed from the deeper implant (ILP 1) to the hub (S-ICD 2) via IBC. The hub (S-ICD 2) supports interfacing with a remote service monitoring infrastructure (external device 3) to enable automated data relay from the "deep" implant's (ILP 1) values/data thereby providing more reliable communication between ILP 1 and the external device 3 and supports a means for accessing automated reimbursable remote follow-up.

## Claims

1. A medical device system for implantation within a patient comprising a first implantable device (1) and a second implantable device (2), wherein the second implantable device (2) is configured to be implanted at a shallow region of the patient's body,
wherein the first implantable device (1) has a first measurement unit (126) configured to record at least one bodily parameter, a first processor (120) configured to process the recorded values of the at least one bodily parameter and a first communication unit (128), wherein the second implantable device (2) has a second data memory and a second communication unit configured to receive recorded values of the at least one bodily parameter and/or data produced by the first processor (120) based on the recorded values, wherein the second data memory is configured to store received recorded values and/or data, wherein the second communication unit is further configured to transmit said recorded values and/or data to an external device (3), wherein the communication between the first communication unit (128) and the second communication unit is an intra-body communication using ultrasonic signals and/or electric signals.

2. The medical device system according to claim 1, wherein first implantable device is an ILP (1) and/or the second implantable device is a hub-like bio-monitor or an ICD (2) and/or the external device (3) is a computer configured for home-monitoring.

3. The medical device system of any of the previous claims, wherein the second implantable device (2) comprises a second measurement unit configured to record values of at least one bodily parameter, wherein the second communication unit is configured to transmit to the external device (3) further recorded values of the at least one bodily parameter by the second measurement unit and/or data produced by a second processor based on said further recorded values.

4. The medical device system of any of the previous claims, wherein said recorded values, said further recorded values and/or said data are ECG data and/or IEGM data.

5. The medical device system of any of the previous claims, wherein the first communication unit (128) and/or the second communication unit is configured to transmit said recorded values, said further recorded values and said data at a pre-defined fixed time interval or a varying time interval, for example if fulfillment of a pre-defined condition is recognized.

6. The medical device system of any of the previous claims, wherein the second communication unit is configured to transmit said recorded values, said data and/or said further recorded values only in case the second measurement unit has detected that the external device is located within a communication range of the second communication unit.

7. The medical device system of any of the previous claims, wherein the first communication unit (128) and/or the second communication unit is configured to transmit said recorded values, said data and/or said further recorded values together with a respective time stamp of the time and/or date of recording.

8. An operation method of a medical device system for implantation within a patient comprising a first implantable device (1) and a second implantable device (2), wherein the second implantable device (2) is configured to be implanted at a shallow region of the patient's body,
wherein a first measurement unit (126) of the first implantable device records at least one bodily parameter, a first processor (120) processes the recorded values of the at least one bodily parameter, wherein a second communication unit of the second implantable device receives recorded values of the at least one bodily parameter and/or data produced by the first processor (120) based on the recorded values transmitted from the first communication unit (128), wherein a second data memory of the second implantable device (2) stores received recorded values and/or data, wherein the second communication unit transmits said recorded values and/or data to an external device, wherein the communication between the first communication unit (128) and the second communication unit is an intra-body communication using ultrasonic signals and/or electric signals.

9. The method of claim 8, wherein a second measurement unit of the second implantable device (2) records values of at least one bodily parameter, wherein the second communication unit transmits to the external device further recorded values of the at least one bodily parameter by the second measurement unit and/or data produced by a second processor based on said further recorded values.

10. The method of any of the claims 8 to 9, wherein said recorded values, said further recorded values and/or said data are ECG data and/or IEGM data.

11. The method of any of the claims 8 to 10, wherein the first communication unit (128) and/or the second communication unit transmits said recorded values, said further recorded values and said data at a pre-defined fixed time interval or a varying time interval, for example if fulfillment of a pre-defined condition is recognized.

12. The method of any of the claims 8 to 11, wherein the second communication unit transmits said recorded values, said data and/or said further recorded values only in case the second measurement unit has detected that the external device is located within a communication range of the second communication unit.

13. The method of any of the claims 8 to 12, wherein the first communication unit (128) and/or the second communication unit transmits said recorded values, said data and/or said further recorded values together with a respective time stamp of the time and/or date of recording.

14. A computer program product comprising instructions which, when executed by a processor, cause the processor to perform the steps of the method according to any of the claims 8 to 13.

15. Computer readable data carrier storing a computer program product according to claim 14.
